# EUROPEAN PATENT APPLICATION

(11) **EP 2 896 412 A1**
(43) Date of publication of application: **22.07.2015**
(21) Application number: 15000183.2
(22) Date of filing: 21.02.2006
(51) Int. Cl.: A61L 31/16, A61L 31/10, A61K 31/436, A61L 29/04, A61L 29/08, A61L 29/16, A61L 31/04

(54) **Drugs with improved hydrophobicity for incorporation in medical devices**

(30) Priority: 18.02.2005 US 654175 P
(62) Divisional of application: 06720876.9
(71) Applicant: Abraxis BioScience, LLC, Los Angeles, CA 90025 (US)
(72) Inventor: Wang, Qinwei, Arcadia, CA 91007 (US); YU, ChengZhi, Los Angeles, CA 90025 (US); Soon-Shiong, Patrick, Los Angeles, CA 90049 (US); Chunlin, Tao, Los Angeles, CA 90025 (US); Desai, Neil P., Los Angeles, CA 90025 (US)
(74) Representative: Grünecker Patent- und Rechtsanwälte PartG mbB

(57) **Abstract**

The invention provides a medical device comprising a hydrophobic analog of a medicament known to inhibit cell proliferation and migration. The invention also provides a method of treating a narrowing in a body passageway comprising placing an implantable medical device comprising a hydrophobic analog of a medicament known to inhibit cell proliferation and migration. The medicaments can be incorporated within or coated on the device. The invention further provides hydrophobic analogs of medicaments known to inhibit cell proliferation and migration.

## Description

### CROSS-REFERENCE TO RELATED APPLICATIONS

This patent application claims the benefit of U.S. Provisional Patent Application No. 60/654,175 filed February 18, 2005.

### FIELD OF THE INVENTION

The present invention relates to delivery devices coated with therapeutically active agents. More particularly, the present invention relates to stents and the like coated with hydrophobic analogs of therapeutically active agents and method of use thereof.

### BACKGROUND OF THE INVENTION

There are many passageways within the body which allow the flow of essential materials. These include, for example, arteries and veins, the esophagus, stomach, small and large intestine, biliary tract, ureter, bladder, urethra, nasal passageways, trachea and other airways, and the male and female reproductive tract. Injury, various surgical procedures, or disease can result in the narrowing, weakening and/or obstruction of such body passageways, resulting in serious complications and/or even death.

Coronary heart disease is the major cause of death in men over the age of 40 and in women over the age of fifty in the western world. Most coronary artery related deaths are due to atherosclerosis. Atherosclerotic lesions which limit or obstruct coronary blood flow are the major cause of ischemic heart disease related mortality and result in 500,000-600,000 deaths in the United States annually. To arrest the disease process and prevent the more advanced disease states in which the cardiac muscle itself is compromised, direct intervention has been employed via percutaneous transluminal coronary angioplasty (PTCA) or coronary artery bypass graft (CABG).

PTCA is a procedure in which a small balloon tipped catheter is passed down a narrowed coronary artery and then expanded to re-open the artery. The major advantage of this therapy is that patients in which the procedure is successful need not undergo the more invasive surgical procedure of coronary artery bypass graft. A major difficulty with PTCA is the problem of post angioplasty closure of the vessel, both immediately after PTCA (acute reocclusion) and in the long term (restenosis).

The mechanism of acute reocclusion appears to involve several factors and may result from vascular recoil with resultant closure of the artery and/or deposition of blood platelets along the damaged length of the newly opened blood vessel followed by formation of a fibrin/red blood cell thrombus. Recently, intravascular stents have been examined as a means of preventing acute reclosure after PTCA. Stents act as scaffoldings, functioning to physically hold open and, if desired, to expand the wall of the passageway. Typically stents are capable of being compressed, so that they can be inserted through small cavities via catheters, and then expanded to a larger diameter once they are at the desired location. Examples in the patent literature disclosing stents that have been applied in PTCA procedures include U.S. Patent No. 4,733,665 issued to Palmaz, U.S. Patent No. 4,800,882 issued to Gianturco, and U.S. Patent No. 4,886,062 issued to Wiktor. Mechanical intervention via stents has reduced the rate of restenosis as compared to balloon angioplasty. Yet, restenosis is still a significant clinical problem with rates ranging from 20-40%. When restenosis does occur in the stented segment, its treatment can be challenging, as clinical options are more limited as compared to lesions that were treated solely with a balloon.

More recently, the solution moved away from the purely mechanical devices and towards a combination of the devices with pharmacologic agents. Sometimes referred to as a "coated" or "medicated" stent, a drug eluting stent is a normal metal stent that has been coated with a pharmacologic agent (drug) that is known to interfere with the process of restenosis. Physicians and companies began testing a variety of drugs that were known to interrupt the biological processes that caused restenosis. The drug eluting stent has been extremely successful in reducing restenosis from the 20-30% range to single digits. Currently two drug eluting stents, the Cordis CYPHER™ sirolimus eluting stent and the Boston Scientific TAXUS™ paclitaxel eluting stent system, have received FDA approval for sale in the United States (the Cypher stent in April 2003; the Taxus™ stent was approved in March 2004) as well as the CE mark for sale in Europe. In addition, the Cook V Flex Plus is available in Europe. Medtronic and Guidant both have drug eluting stent programs in the early stages of clinical trials and are looking to 2005 or 2006 for possible approval.

### Mechanism of Restenosis

In the normal arterial wall, smooth muscle cells (SMC) proliferate at a low rate (<0.1%/day; ref). SMC in vessel wall exists in a 'contractile' phenotype characterized by 80 to 90% of the cell cytoplasmic volume occupied with the contractile apparatus. Endoplasmic reticulum, golgi bodies, and free ribosomes are few and are located in the perinuclear region. Extracellular matrix surrounds SMC and is rich in heparin like glycosylaminoglycans which are believed to be responsible for maintaining SMC in the contractile phenotypic state.

Upon pressure expansion of an intracoronary balloon catheter during angioplasty/stenting, endothelial cells and smooth muscle cells within the arterial wall become injured. Cell derived growth factors, for example, platelet derived growth factor (PDGF), basic fibroblast growth factor (bFGF), epidermal growth factor (EGF), etc., which are released from platelets (e.g., PDGF) adhering to the damaged arterial luminal surface, invading macrophages and/or leukocytes, or directly from SMC (e.g., BFGF), provoke a proliferation and migratory response in medial SMC. These cells undergo a phenotypic change from the contractile phenotyope to a synthetic phenotype characterized by only few contractile filament bundles, but extensive rough endoplasmic reticulum, golgi, and free ribosomes. Proliferation/migration usually begins within 1-2 days post injury and peaks at 2 days in the media, rapidly declining thereafter (Campbell et al., in Vascular Smooth Muscle Cells in Culture, Campbell, J. H. and Campbell, G. R., Eds, CRC Press, Boca Raton, 1987, pp. 39-55); Clowes, A. W. and Schwartz, S. M., Circ. Res. 56:139-145, 1985).

Daughter synthetic cells migrate to the intimal layer of arterial smooth muscle and continue to proliferate. Proliferation and migration continues until the damaged luminal endothelial layer regenerates at which time proliferation ceases within the intima, usually within 7-14 days postinjury. The remaining increase in intimal thickening which occurs over the next 3-6 months is due to an increase in extracellular matrix rather than cell number. Thus, SMC migration and proliferation is an acute response to vessel injury while intimal hyperplasia is a more chronic response. (Liu et al., Circulation, 79:1374-1387, 1989).

### Use of Stenting in Non Vascular Applications

Many types of tumors (both benign and malignant) can result in damage to the wall of a body passageway or obstruction of the lumen, thereby slowing or preventing the flow of materials through the passageway. Obstruction in body passageways that are affected by cancer are not only in and of themselves life threatening, they also limit the quality of a patient's life.

The primary treatment for the majority of tumors which cause neoplastic obstruction is surgical removal and/or chemotherapy, radiation therapy, or laser therapy. Unfortunately, by the time a tumor causes an obstruction in a body passageway it is frequently inoperable and generally will not respond to traditional therapies. One approach to this problem has been the insertion of endoluminal stents. However, a significant drawback to the use of stents in neoplastic obstruction is that the tumor is often able to grow into the lumen through the interstices of the stent. In addition, the presence of a stent in the lumen can induce the ingrowth of reactive or inflammatory tissue (e.g., blood vessels, fibroblasts and white blood cells) onto the surface of the stent. If this ingrowth (composed of tumor cells and/or inflammatory cells) reaches the inner surface of the stent and compromises the lumen, the result is re-blockage of the body passageway which the stent was inserted to correct.

Other diseases, which although not neoplastic, nevertheless involve proliferation, can likewise obstruct body passageways. For example, narrowing of the prostatic urethra due to benign prostatic hyperplasia is a serious problem affecting 60% of all men over the age of 60 years of age and 100% of all men over the age of 80 years of age. Present pharmacological treatments, such as 5α-reductase inhibitors (e.g., Finasteride^{®}), or α-adrenergic blockers (e.g., Terazozan^{®}) are generally only effective in a limited population of patients.

Moreover, of the surgical procedures that can be performed (e.g., trans-urethral resection of the prostate (TURPs); open prostatectomy, or endo-urologic procedures such as laser prostatectomy, use of microwaves, hypothermia, cryosurgery, or stenting), numerous complications such as bleeding, infection, incontinence, impotence, and recurrent disease, typically result.

### BRIEF SUMMARY OF THE INVENTION

The invention provides a medical device comprising a hydrophobic analog of a medicament known to inhibit cell proliferation and migration. Examples of suitable medicaments include, for example, geldanamycin antibiotics, colchicines, combrestatins, camptothecins, taxanes, and rapamycin, and analogs thereof. The medicaments can be incorporated within or coated on the device.

The invention also provides a method of treating a narrowing in a body passageway comprising placing an implantable medical device comprising a hydrophobic analog of a medicament known to inhibit cell proliferation and migration. Examples of suitable medicaments include, for example, geldanamycin antibiotics, colchicines, combrestatins, camptothecins, taxanes, and rapamycin and analogs thereof. The medicaments can be incorporated within or coated on the device.

The invention further provides hydrophobic analogs of medicaments known to inhibit cell proliferation and migration. Examples of suitable medicaments include, for example, geldanamycin antibiotics, colchicines, combrestatins, camptothecins, taxanes, rapamycin, and analogs thereof. The medicaments can be incorporated within or coated on the device.

In another embodiment, the invention provides a medical device comprising a medicament comprising a hydrophobic analog of a taxane of the formula:

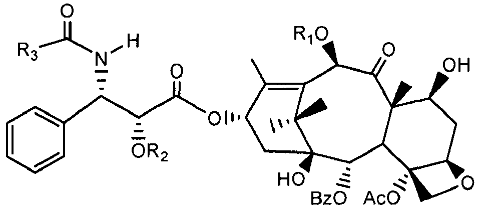

wherein, R₁ is H or Ac; R₂ is H, COPh or CO(CH₂)₄CH₃; and R₃ is Ph or OtBu, wherein the analog of a taxane is not paclitaxel or docetaxel.

In another embodiment, the invention provides a medical device comprising a medicament comprising a hydrophobic analog of a taxane of the formula: 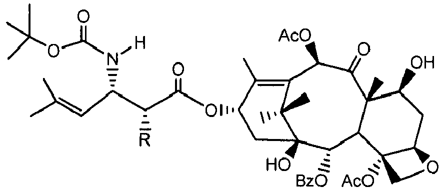 wherein R is OH, OCOPh or OCO(CH₂)₄CH₃.
or 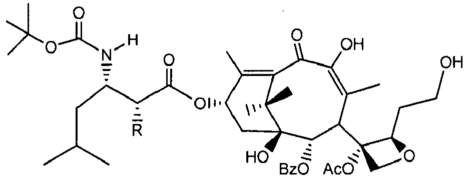 wherein R is OH, OCOPh or OCO(CH₂)₄CH₃.

In another embodiment, the invention provides a medical device comprising a medicament comprising camptothecin or a hydrophobic analog of a camptothecin of the formula: 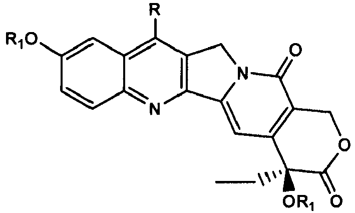 wherein, R is H, methyl, or ethyl; and R₁ is H or CO(X), wherein X is C₂-C₁₈ alkyl, phenyl, CH₂NHCO₂tBu CH₂OMe, CH₂NH₂.

In yet another embodiment, the invention provides a medical device comprising a medicament comprising rapamycin or a hydrophobic analog of rapamycin of the formula: 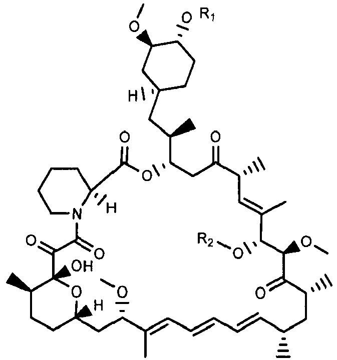 wherein, R₁ is H and R₂ is H or COPh.

In another embodiment, the invention provides a medical device comprising a a hydrophobic dimer of the formula: 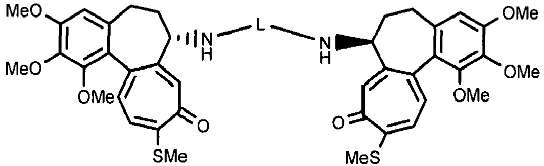 wherein L is:
(a) 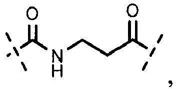
(b) 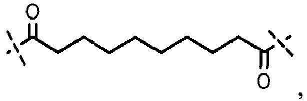
(c) 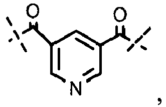 or
(d)

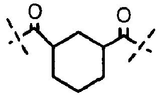

In a further embodiment, the invention provides a medical device comprising a medicament comprising geldanamycin or a hydrophobic analog of geldanamycin of the formula: 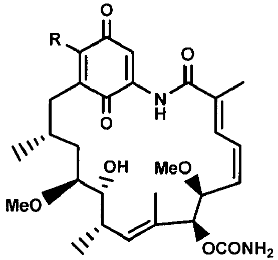 wherein, R is OMe, NHCHCH₂, NH(CH₂)₆CH₃, N(CH₂)₅, NCH₂CHCH₃, or NHCH(CH₃)(CH₂)₄CH₃.

In yet another embodiment, the invention provides a medical device comprising a medicament comprising combretastatin or a hydrophobic analog of combretastatin of the formula: 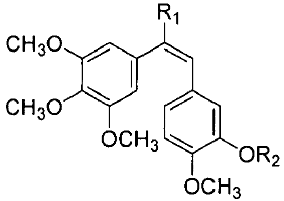
(a): R₁ is H; R₂ is H
(b): R₁ is CO₂H; R₂ is H
(c): R₁ is CO₂H; R₂ is COCH₃
(d): R₁ is H; R₂ is COCH₃
(e): R₁ is H; R₂ is CO(CH₂)₄CH₃
(f): R₁ is H; R₂ is CO(CH₂)₁₀CH₃
(g): R₁ is H; R₂ is CO(CH₂)₆(CH₂CH=CH)₂(CH₂)₄CH₃
(h): R₁ is H; R₂ is CO(CH₂)₇CH=CH(CH₂)₇CH₃

### EMBODIMENTS OF THE INVENTION

The present invention provides in embodiments:
1. A medical device comprising a medicament comprising a hydrophobic analog of a taxane of the formula: 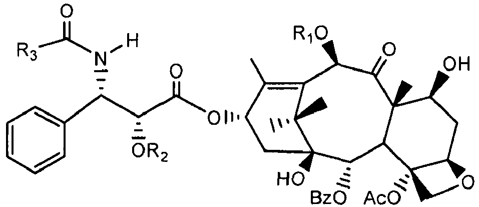 wherein, R₁ is H or Ac; R₂ is H, COPh or CO(CH₂)₄CH₃; and R₃ is Ph or OtBu, wherein the analog of a taxane is not paclitaxel or docetaxel.
2. The device of embodiment 1, wherein said medicament is coated on or incorporated within the body of the device.
3. The device of embodiment 1, wherein the medicament is incorporated onto or within the device in presence of a polymer.
4. The device of embodiment 2, wherein said analog is present in the coating in an amount of from about 0.0001% to about 30% by weight of said coating.
5. The device of embodiment 3, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
6. The device of embodiment 3, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
7. The device of embodiment 6, wherein said medicament is coated on or incorporate within the body of the device.
8. The device of embodiment 6, wherein the medicament is incorporated onto or within the device in presence of a polymer.
9. The device of embodiment 7, wherein said analog is present in the coating in an amount of from about 0.0001% to about 30% by weight of said coating.
10. The device of embodiment 8, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
11. The device of embodiment 8, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
12. A medical device comprising a medicament comprising a hydrophobic analog of a taxane of the formula: 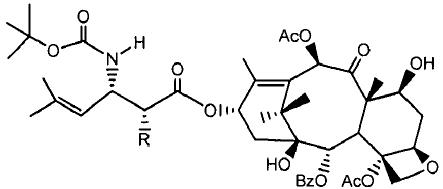 wherein R is OH, OCOPh or OCO(CH₂)₄CH₃.
   or 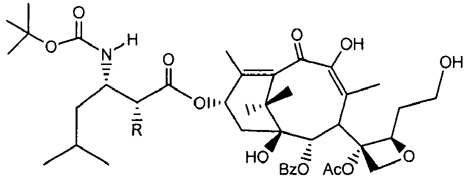 wherein R is OH, OCOPh or OCO(CH₂)₄CH₃.
13. The device of embodiment 12, wherein said medicament is coated on or incorporated within the body of the device.
14. The device of embodiment 12, wherein the medicament is incorporated onto or within the device in presence of a polymer.
15. The device of embodiment 13, wherein said analog is present in the coating in an amount of from about 0.0001% to about 30% by weight of said coating.
16. The device of embodiment 14, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
17. The device of embodiment 14, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
18. The device of embodiment 17, wherein said medicament is coated on or incorporate within the body of the device.
19. The device of embodiment 17, wherein the medicament is incorporated onto or within the device in presence of a polymer.
20. The device of embodiment 18, wherein said analog is present in the coating in an amount of from about 0.0001% to about 30% by weight of said coating.
21. The device of embodiment 19, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
22. The device of embodiment 19, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
23. A medical device comprising a medicament comprising camptothecin or a hydrophobic analog of a camptothecin of the formula: 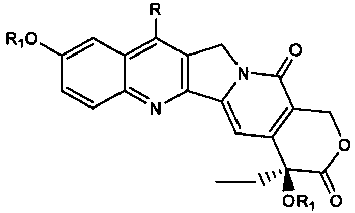 wherein, R is H, methyl, or ethyl; and R₁ is H or CO(X), wherein X is C₂-C₁₈ alkyl, phenyl, CH₂NHCO₂tBu CH₂OMe, CH₂NH₂.
24. The device of embodiment 23, wherein said medicament is coated on or incorporated within the body of the device.
25. The device of embodiment 23, wherein the medicament is incorporated onto or within the device in presence of a polymer.
26. The device of embodiment 24, wherein said analog is present in the coating in an amount of from about 0.0001% to about 30% by weight of said coating.
27. The device of embodiment 25, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
28. The device of embodiment 25, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
29. The device of embodiment 28, wherein said medicament is coated on or incorporate within the body of the device.
30. The device of embodiment 28, wherein the medicament is incorporated onto or within the device in presence of a polymer.
31. The device of embodiment 29, wherein said analog is present in the coating in an amount of from about 0.0001% to about 30% by weight of said coating.
32. The device of embodiment 30, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
33. The device of embodiment 30, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
34. A medical device comprising a medicament comprising rapamycin or a hydrophobic analog of rapamycin of the formula: 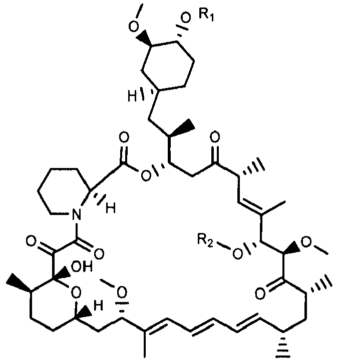 wherein, R₁ is H and R₂ is H or COPh.
35. The device of embodiment 34, wherein said medicament is coated on or incorporated within the body of the device.
36. The device of embodiment 34, wherein the medicament is incorporated onto or within the device in presence of a polymer.
37. The device of embodiment 35, wherein said analog is present in the coating in an amount of from about 0.0001% to about 30% by weight of said coating.
38. The device of embodiment 36, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
39. The device of embodiment 36, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
40. The device of embodiment 39, wherein said medicament is coated on or incorporate within the body of the device.
41. The device of embodiment 39, wherein the medicament is incorporated onto or within the device in presence of a polymer.
42. The device of embodiment 40, wherein said analog is present in the coating in an amount of from about 0.0001% to about 30% by weight of said coating.
43. The device of embodiment 41, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
44. The device of embodiment 41, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
45. A medical device comprising a medicament comprising a dimer of the formula: 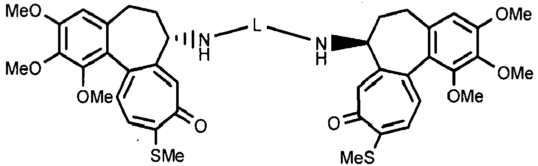 wherein L is:
   (a) 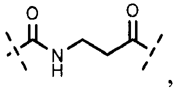
   (b) 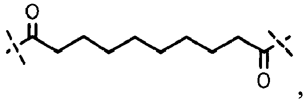
   (c) 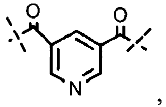 or
   (d) 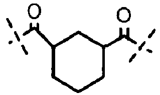
46. The device of embodiment 45, wherein said medicament is coated on or incorporated within the body of the device.
47. The device of embodiment 45, wherein the medicament is incorporated onto or within the device in presence of a polymer.
48. The device of embodiment 46, wherein said analog is present in the coating in an amount of from about 0.0001% to about 30% by weight of said coating.
49. The device of embodiment 47, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
50. The device of embodiment 47, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
51. The device of embodiment 50, wherein said medicament is coated on or incorporate within the body of the device.
52. The device of embodiment 50, wherein the medicament is incorporated onto or within the device in presence of a polymer.
53. The device of embodiment 51, wherein said analog is present in the coating in an amount of from about 0.0001% to about 30% by weight of said coating.
54. The device of embodiment 52, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
55. The device of embodiment 52, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
56. A medical device comprising a medicament comprising geldanamycin or a hydrophobic analog of a geldanamycin of the formula: 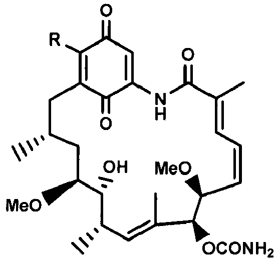 wherein, R is OMe, NHCHCH₂, NH(CH₂)₆CH₃, N(CH₂)₅, NCH₂CHCH₃, or NHCH(CH₃)(CH₂)₄CH₃.
57. The device of embodiment 56, wherein said medicament is coated on or incorporated within the body of the device.
58. The device of embodiment 56, wherein the medicament is incorporated onto or within the device in presence of a polymer.
59. The device of embodiment 57, wherein said analog is present in the coating in an amount of from about 0.0001% to about 30% by weight of said coating.
60. The device of embodiment 58, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
61. The device of embodiment 58, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
62. The device of embodiment 61, wherein said medicament is coated on or incorporate within the body of the device.
63. The device of embodiment 61, wherein the medicament is incorporated onto or within the device in presence of a polymer.
64. The device of embodiment 62, wherein said analog is present in the coating in an amount of from about 0.0001% to about 30% by weight of said coating.
65. The device of embodiment 63, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
66. The device of embodiment 63, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
67. A medical device comprising a medicament comprising combretastatin or a hydrophobic analog of a combretastatin of the formula: 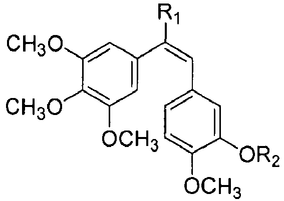 wherein
   (a): R₁ is H; R₂ is H
   (b): R₁ is CO₂H; R₂ is H
   (c): R₁ is CO₂H; R₂ is COCH₃
   (d): R₁ is H; R₂ is COCH₃
   (e): R₁ is H; R₂ is CO(CH₂)₄CH₃
   (f): R₁ is H; R₂ is CO(CH₂)₁₀CH₃
   (g): R₁ is H; R₂ is CO(CH₂)₆(CH₂CH=CH)₂(CH₂)₄CH₃ (h): R₁ is H; R₂ is CO(CH₂)₇CH=CH(CH₂)₇CH₃
68. The device of embodiment 67, wherein said medicament is coated on or incorporated within the body of the device.
69. The device of embodiment 67, wherein the medicament is incorporated onto or within the device in presence of a polymer.
70. The device of embodiment 68, wherein said analog is present in the coating in an amount of from about 0.0001% to about 30% by weight of said coating.
71. The device of embodiment 69, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
72. The device of embodiment 69, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
73. The device of embodiment 72, wherein said medicament is coated on or incorporate within the body of the device.
74. The device of embodiment 72, wherein the medicament is incorporated onto or within the device in presence of a polymer.
75. The device of embodiment 73, wherein said analog is present in the coating in an amount of from about 0.0001% to about 30% by weight of said coating.
76. The device of embodiment 74, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.
77. The device of embodiment 74, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.

### DETAILED DESCRIPTION OF THE INVENTION

### Drug Hydrophobicity And Effectiveness

The invention provides a medical device and method of treating a narrowing in a body passageway comprising placing a medical device, comprising a hydrophobic analog of a medicament known to inhibit cell proliferation and migration, into a body passageway. Medical devices of the invention include, but are not limited to, a stent, a catheter, a balloon, a wire guide, a cannula, central line, vascular valves, prosthetics for treatment of aneurysms, and the like.

Currently, stents coated with paclitaxel and rapamycin (sirolimus) are approved for use in coronary PTCA procedures and are useful in reducing the rate of restenosis compared to bare metal stents. While the focus of stent companies has been on pursuing these and other compounds that may be effective in reducing hyperplasia and restenosis at the stent site, what is not readily recognized is that the hydrophobicity of the drug plays an important role in the penetration, persistence, retention, and effectiveness of the drug in the tissue, for example, a blood vessel, once it is released from the device, for example an intravascular stent.

The compounds of the present invention are new analogs or prodrugs of known parent compounds and aim to increase hydrophobicity as compared to their parent compounds of known cytotoxic activity, for example, paclitaxel, docetaxel, rapamycin (sirolimus), geldanamycin, colchicine, combretastatin, and the like. It was surprisingly determined that the hydrophobic analogs of compounds bind to cellular components in blood vessels, for example, proteins, cell membranes, etc., with greater affinity dependent on their hydrophobicity. Such compounds have use in devices such as intravascular devices, including as stents, where the released drug must persist in the vessel wall to prevent or reduce the incidence of restenosis.

In another aspect, compounds that inhibit the epidermal growth factor receptor (EGFR) are found to be useful for prevention of proliferation and migration of cells and useful for treatment of restenosis.

### Role Of EGF Receptor In Proliferation And Migration Of Cells

Targeting genistein (Gen) (5,7,4' trihydroxyisoflavone), a naturally occurring tyrosine kinase inhibitor present in soybeans (Aikyama et al., 1987, J. Biol. Chem, 262:5592-5595; Uckun et al., 1995, Science 267:886-891), to the EGF receptor/PTK complexes in breast cancer cells using the EGF Gen conjugate resulted in marked inhibition of the EGF receptor tyrosine kinase and EGF receptor associated Src family PTK (Uckun et al., 1998, Clinical Cancer Research, 4: 901-912).

Proliferating vascular smooth muscle cells also express high levels of the EGF receptor (Saltis et al., 1995, Atherosclerosis, 118:77-87). Furthermore, a noninvasive small animal model of restenosis, which employs photoactivated rose bengal to induce vascular injury to the femoral arteries of C57B 1/6 mice leading to neointimal hyperplasia mimicking the post PTCA restenosis of coronary arteries, demonstrated that the myofibroblasts of the neointima were EGF receptor positive in 8 of 8 mice (100%) analyzed (Trieu et al, 2000, J. Cardiovasc. Pharmacology, 35: 595-605). Notably, the neointima of the injured femoral arteries stained more intensely with the anti EGF receptor antibody than the media and/or intima of uninjured femoral arteries (Trieu et al., 2000, J. Cardiovasc. Pharmacology, 35: 595-605). In a proof of concept experiment, EGF genistein was shown to be effective in this mouse model of restenosis (Trieu et al., 2000, J. Cardiovasc. Pharmacology, 35: 595-605).

These findings suggest that the EGF receptor function and EGF receptor linked signal transduction events may be essential for the migration and proliferation of myofibroblasts contributing to the neointimal hyperplasia after vascular injury. It was then postulated that the EGF receptor on vascular smooth muscle cells may be a suitable target for restenosis prophylaxis using EGF receptor directed tyrosine kinase inhibitors. Recently the multichaperone heat shock protein (Hsp) 90 has been shown to mediate the maturation and stability of a variety of proteins including EGF R (Zhang et al. (2004) J. Mol. Med. 82: 488-499.). Compounds of the present invention, especially the derivatives and analogs of geldanamycin are effective inhibitors of HSP 90 and therefore are useful in reducing proliferation and migration of cells and in treatment of restenosis.

### Polymers And Coating Of Devices

Loading of drugs on a stent or other suitable medical device may be achieved by any number of methods, such as those described by Hossainy et al. (U.S. Patent 6,153,252).

Film forming polymers that can be used for coatings in this application can be absorbable or non absorbable and must be biocompatible to minimize irritation to the vessel wall. The polymer may be either biostable or bioabsorbable depending on the desired rate of release or the desired degree of polymer stability, but a bioabsorbable polymer is preferred since, unlike biostable polymer, it will not be present long after implantation to cause any adverse, chronic local response. Furthermore, bioabsorbable polymers do not present the risk that over extended periods of time there could be an adhesion loss between the stent and coating caused by the stresses of the biological environment that could dislodge the coating and introduce further problems even after the stent is encapsulated in tissue.

Suitable film forming bioabsorbable polymers that could be used include polymers selected from the group consisting of aliphatic polyesters, poly(amino acids), copoly(ether esters), polyalkylenes oxalates, polyamides, poly(iminocarbonates), polyorthoesters, polyoxaesters, polyamidoesters, polyoxaesters containing amido groups, poly(anhydrides), polyphosphazenes, biomolecules and blends thereof. For the purpose of this invention, aliphatic polyesters include homopolymers and copolymers of lactide (which includes lactic acid D-,L-and *meso*-lactide), ε-caprolactone, glycolide (including glycolic acid), hydroxybutyrate, hydroxyvalerate, *para*-dioxanone, trimethylene carbonate (and its alkyl derivatives), 1,4-dioxepan 2 one, 1,5-dioxepan 2 one, 6,6-dimethyl 1,4-dioxin-2-one and polymer blends thereof. Poly(iminocarbonate), for the purpose of this invention, include those as described by Kemnitzer and Kohn, in the Handbook of Biodegradable Polymers, edited by Domb, Kost and Wisemen, Hardwood Academic Press, 1997, pages 251-272. Copoly(ether esters) for the purpose of this invention include those copolyester ethers described in Journal of Biomaterials Research, vol. 22, pages 993-1009, 1988 by Cohn and Younes and Cohn, Polymer Preprints (ACS Division of Polymer Chemistry) vol. 30(1), page 498, 1989 (e.g. PEO/PLA). Polyalkylene oxalates for the purpose of this invention include U.S. Patents 4,208,511; 4,141,087; 4,130,639; 4,140,678; 4,105,034; and 4,205,399, which are incorporated by reference herein. Polyphosphazenes, co-, ter-, and higher order mixed monomer based polymers made from L lactide, D,L lactide, lactic acid, glycolide, glycolic acid, *para*-dioxanone, trimethylene carbonate and ε-caprolactone such as are described by Allcock in The Encyclopedia of Polymer Science, vol. 13, pages 31-41, Wiley Intersciences, John Wiley & Sons, 1988 and by Vandorpe, Schacht, Dejardin and Lemmouchi in the Handbook of Biodegradable Polymers, edited by Domb, Kost and Wisemen, Hardwood Academic Press, 1997, pages 161-182 (which are hereby incorporated by reference herein). Polyanhydrides from diacids of the form HOOC C₆H₄-O-(CH₂)ₘ-O-C₆H₄-COOH where m is an integer in the range of from 2 to 8 and copolymers thereof with aliphatic alpha omega diacids of up to 12 carbons. Polyoxaesters polyoxaamides and polyoxaesters containing amines and/or amido groups are described in one or more of the following U.S. Patents 5,464,929; 5,595,751; 5,597,579; 5,607,687; 5,618,552; 5,620,698; 5,645,850; 5,648,088; 5,698,213 and 5,700,583; (which are incorporated herein by reference). Polyorthoesters such as those described by Heller in Handbook of Biodegradable Polymers, edited by Domb, Kost and Wisemen, Hardwood Academic Press, 1997, pages 99-118 (which is hereby incorporated herein by reference). Film forming polymeric biomolecules for the purpose of this invention include naturally occurring materials that may be enzymatically degraded in the human body or are hydrolytically unstable in the human body such as fibrin, fibrinogen, collagen, elastin, and absorbable biocompatable polysaccharides such as chitosan, starch, fatty acids (and esters thereof), glucoso glycans and hyaluronic acid.

Suitable film forming biostable polymers with relatively low chronic tissue response, such as polyurethanes, silicones, poly(meth)acrylates, polyesters, polyalkyl oxides (polyethylene oxide), polyvinyl alcohols, polyethylene glycols and polyvinyl pyrrolidone, as well as, hydrogels such as those formed from crosslinked polyvinyl pyrrolidinone and polyesters could also be used. Other polymers could also be used if they can be dissolved, cured or polymerized on the stent. These include polyolefins, polyisobutylene and ethylene alphaolefin copolymers; acrylic polymers (such as methacrylate) and copolymers, vinyl halide polymers and copolymers, such as polyvinyl chloride; polyvinyl ethers, such as polyvinyl methyl ether; polyvinylidene halides such as polyvinylidene fluoride and polyvinylidene chloride; polyacrylonitrile, polyvinyl ketones; polyvinyl aromatics such as polystyrene; polyvinyl esters such as polyvinyl acetate; copolymers of vinyl monomers with each other and olefins, such as etheylene methyl methacrylate copolymers, acrylonitrile styrene copolymers, ABS resins and ethylene vinyl acetate copolymers; polyamides, such as Nylon 66 and polycaprolactam; alkyd resins; polycarbonates; polyoxymethylenes; polyimides; polyethers; epoxy resins, polyurethanes; rayon; rayon triacetate, cellulose, cellulose acetate, cellulose acetate butyrate; cellophane; cellulose nitrate; cellulose propionate; cellulose ethers (e.g., carboxymethyl cellulose and hydoxyalkyl celluloses); and combinations thereof. Polyamides for the purpose of this application would also include polyamides of the form NH(CH2)-CO and NH (CH2)x-NH-CO-(CH2)_{y}-CO, wherein n is preferably an integer in from 6 to 13; x is an integer in the range of form 6 to 12; and y is an integer in the range of from 4 to 16. The list provided above is illustrative but not limiting.

The polymers used for coatings must be film forming polymers that have a molecular weight high enough as to not be waxy or tacky. The polymers also must adhere to the stent and not be so readily deformable after deposition on the stent as to be able to be displaced by hemodynamic stresses. The polymers molecular weight should be high enough to provide sufficient toughness so that the polymers will not to be rubbed off during handling or deployment of the stent and must not crack during expansion of the stent. The melting point of the polymer used in the present invention should have a melting temperature above 40 °C, preferably above about 45 °C, more preferably above 50 °C and most preferably above 55 °C.

The preferable coatings to use for this application are bioabsorbable elastomers, more preferably aliphatic polyester elastomers. In the proper proportions aliphatic polyester copolymers are elastomers. Elastomers present the advantage that they tend to adhere well to the metal stents and can withstand significant deformation without cracking. The high elongation and good adhesion provide superior performance to other polymer coatings when the coated stent is expanded. Examples of suitable bioabsorbable elastomers are described in U.S. Patent 5,468,253, which is hereby incorporated by reference. Preferably the bioabsorbable biocompatible elastomers based on aliphatic polyester, including but not limited to those selected from the group consisting of elastomeric copolymers of ε-caprolactone and glycolide (preferably having a mole ratio of ε-caprolactone to glycolide of from about 35:65 to about 65:35, more preferably 45:55 to 35:65) elastomeric copolymers of ε-caprolactone and lactide, including L-lactide, D-lactide blends thereof or lactic acid copolymers (preferably having a mole ratio of ε-caprolactone to lactide of from about 35:65 to about 90:10 and more preferably from about 35:65 to about 65:35 and most preferably from about 45:55 to 30:70 or from about 90:10 to about 80:20) elastomeric copolymers of *p-*dioxanone (1,4-dioxin-2-one) and lactide including L-lactide, D-lactide and lactic acid (preferably having a mole ratio of *p*-dioxanone to lactide of from about 40:60 to about 60:40) elastomeric copolymers of ε-caprolactone and *p*-dioxanone (preferably having a mole ratio of ε-caprolactone to *p*-dioxanone of from about 30:70 to about 70:30) elastomeric copolymers of *p*-dioxanone and trimethylene carbonate (preferably having a mole ratio of *p*-dioxanone to trimethylene carbonate of from about 30:70 to about 70:30), elastomeric copolymers of trimethylene carbonate and glycolide (preferably having a mole ratio of trimethylene carbonate to glycolide of from about 30:70 to about 70:30), elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof. As is well known in the art these aliphatic polyester copolymers have different hydrolysis rates, therefore, the choice of elastomer may in part be based on the requirements for the coatings adsorption. For example, ε-caprolactone co-glycolide copolymer (45:55 mole percent, respectively) films lose 90% of their initial strength after 2 weeks in simulated physiological buffer whereas the ε-caprolactone co-lactide copolymers (40:60 mole percent, respectively) loses all of its strength between 12 and 16 weeks in the same buffer. Mixtures of the fast hydrolyzing and slow hydrolyzing polymers can be used to adjust the time of strength retention.

The preferred bioabsorbable elastomeric polymers should have an inherent viscosity of from about 1.0 dL/g to about 4 dL/g, preferably an inherent viscosity of from about 1.0 dL/g to about 2 dL/g and most preferably an inherent viscosity of from about 1.2 dL/g to about 2 dL/g as determined at 25 °C in a 0.1 gram per deciliter (g/dL) solution of polymer in hexafluoroisopropanol (HFIP).

The solvent is chosen such that there is the proper balance of viscosity, deposition level of the polymer, solubility of the pharmaceutical agent, wetting of the stent and evaporation rate of the solvent to properly coat the stents. In the preferred embodiment, the solvent is chosen such that the pharmaceutical agent and the polymer are both soluble in the solvent. In some cases, the solvent must be chosen such that the coating polymer is soluble in the solvent and such that pharmaceutical agent is dispersed in the polymer solution in the solvent. In that case, the solvent chosen must be able to suspend small particles of the pharmaceutical agent without causing them to aggregate or agglomerate into collections of particles that would clog the slots of the stent when applied. Although the goal is to dry the solvent completely from the coating during processing, it is a great advantage for the solvent to be non toxic, non carcinogenic and environmentally benign. Mixed solvent systems can also be used to control viscosity and evaporation rates. In all cases, the solvent must not react with or inactivate the pharmaceutical agent or react with the coating polymer. Preferred solvents include by are not limited to: acetone, *N-*methylpyrrolidone (NMP), dimethyl sulfoxide (DMSO), toluene, methylene chloride, chloroform, 1,1,2-trichloroethane (TCE), various freons, dioxane, ethyl acetate, tetrahydrofuran (THF), dimethylformamide (DMF), and dimethylacetamide (DMAC).

The film forming biocompatible polymer coatings are generally applied to reduce local turbulence in blood flow through the stent, as well as, adverse tissue reactions. The coating may also be used to administer a pharmaceutically active material to the site of the stents placement. Generally, the amount of polymer coating to be placed on the stent will vary with the polymer and the stent design and the desired effect of the coating. As a guideline the amount of coating may range from about 0.5 to about 20 as a percent of the total weight of the stent after coating and preferably will range from about 1 to about 15 percent. The polymer coatings may be applied in one or more coating steps depending on the amount of polymer to be applied. Different polymers may also be used for different layers in the stent coating. In fact, it is highly advantageous to use a dilute first coating solution as primer to promote adhesion of a subsequent coating layer that may contain pharmaceutically active materials.

Additionally, a top coating can be applied to delay release of the pharmaceutical agent, or they could be used as the matrix for the delivery of a different pharmaceutically active material. The amount of top coatings on the stent may vary, but will generally be less than about 2000 µg preferably the amount of top coating will be in the range of about 10 µg to about 1700 µg and most preferably in the range of from about 300 µg to about 1600 µg. Layering of coating of fast and slow hydrolyzing copolymers can be used to stage release of the drug or to control release of different agents placed in different layers. Polymer blends may also be used to control the release rate of different agents or to provide desirable balance of coating (e.g., elasticity, toughness, etc.) and drug delivery characteristics (e.g., release profile). Polymers with different solubilities in solvents can be used to build up different polymer layers that may be used to deliver different drugs or control the release profile of a drug. For example since ε-caprolactone co-lactide elastomers are soluble in ethyl acetate and ε-caprolactone co-glycolide elastomers are not soluble in ethyl acetate. A first layer of ε-caprolactone co-glycolide elastomer containing a drug can be over coated with ε-caprolactone co-glycolide elastomer using a coating solution made with ethyl acetate as the solvent. Additionally, different monomer ratios within a copolymer, polymer structure or molecular weights may result in different solubilities. For example, 45/55 ε-caprolactone co glycolide at room temperature is soluble in acetone whereas a similar molecular weight copolymer of 35/65 ε-caprolactone co-glycolide is substantially insoluble within a 4 weight percent solution. The second coating (or multiple additional coatings) can be used as a top coating to delay the drug deliver of the drug contained in the first layer. Alternatively, the second layer could contain a different drug to provide for sequential drug delivery. Multiple layers of different drugs could be provided by alternating layers of first one polymer then the other. As will be readily appreciated by those skilled in the art numerous layering approaches can be used to provide the desired drug delivery.

### Coating

Coating may be formulated by mixing one or more therapeutic agents with the coating polymers in a coating mixture. The therapeutic agent may be present as a liquid, a finely divided solid, or any other appropriate physical form. Optionally, the mixture may include one or more additives, e.g., nontoxic auxiliary substances such as diluents, carriers, excipients, stabilizers or the like. Other suitable additives may be formulated with the polymer and pharmaceutically active agent or compound. For example, hydrophilic polymers selected from the previously described lists of biocompatible film forming polymers may be added to a biocompatible hydrophobic coating to modify the release profile (or a hydrophobic polymer may be added to a hydrophilic coating to modify the release profile). One example would be adding a hydrophilic polymer selected from the group consisting of polyethylene oxide, polyvinyl pyrrolidone, polyethylene glycol, carboxylmethyl cellulose, hydroxymethyl cellulose and combination thereof to an aliphatic polyester coating to modify the release profile. Appropriate relative amounts can be determined by monitoring the *in vitro* and/or *in vivo* release profiles for the therapeutic agents.

The best conditions for the coating application are when the polymer and pharmaceutical agent have a common solvent. This provides a wet coating that is a true solution. Less desirable, yet still usable are coatings that contain the medicament as a solid dispersion in a solution of the polymer in solvent. Under the dispersion conditions, care must be taken to ensure that the particle size of the dispersed pharmaceutical powder, both the primary powder size and its aggregates and agglomerates, is small enough not to cause an irregular coating surface or to clog the slots of the stent that we need to keep coating free. In cases where a dispersion is applied to the stent and we want to improve the smoothness of the coating surface or ensure that all particles of the drug are fully encapsulated in the polymer, or in cases where we may want to slow the release rate of the drug, deposited either from dispersion or solution, we can apply a clear (polymer only) top coat of the same polymer used to provide sustained release of the drug or another polymer that further restricts the diffusion of the drug out of the coating. The top coat can be applied by dip coating with mandrel as previously described or by spray coating (loss of coating during spray application is less problematic for the clear topcoat since the costly drug is not included). Dip coating of the top coat can be problematic if the drug is more soluble in the coating solvent than the polymer and the clear coating redissolves previously deposited drug. The time spent in the dip bath may need to be limited so that the drug is not extracted out into the drug free bath. Drying should be rapid so that the previously deposited drug does not completely diffuse into the topcoat. A polymer/drug mixture is applied to the surfaces of the stent by either dip coating, or spray coating, or brush coating or dip/spin coating or combinations thereof, and the solvent allowed to evaporate to leave a film with entrapped drug within the polymer.

The amount of therapeutic agent in the coating of the medical device will be dependent upon the particular drug employed, the medical device which includes the therapeutic agent, and the medical condition being treated. Typically, the amount of therapeutic agent represents about 0.0001 % to about 70%, more typically about 0.0001 % to about 60%, most typically about 0.0001% to about 45% by weight of the coating. Lower amounts of therapeutic agent can also be used, such as, for example, from about 0.0001% to about 30% by weight of the coating.

Polymers are biocompatible (e.g., not elicit any negative tissue reaction or promote mural thrombus formation) and degradable, such as lactone-based polyesters or copolyesters, e.g., polylactide, polycaprolactone-glycolide, polyorthoesters, polyanhydrides; poly-aminoacids; polysaccharides; polyphosphazenes; poly(ether-ester) copolymers, e.g., PEO PLLA, or blends thereof. Nonabsorbable biocompatible polymers are also suitable candidates. Polymers such as polydimethyl siloxane; poly(ethylene-vinylacetate); acrylate based polymers or copolymers, e.g., poly(hydroxyethyl methylmethacrylate, polyvinyl pyrrolidinone; polyurethanes; fluorinated polymers such as polytetrafluoroethylene; cellulose esters and copolymers of any of the above polymers are also suitable. In general, polymers described in the art for coating onto medical devices are suitable for this application.

### Coating Without Polymer

Polymers may not always be needed as in the case of devices such as stents, whose body has been modified to contain micropores or channels are dipped into a solution of the therapeutic agent, range 0.001 wt % to saturated, in organic solvent such as acetone, methylene chloride, or other solvent for sufficient time to allow solution to permeate into the pores. The dipping solution can also be compressed to improve the loading efficiency. After solvent has been allowed to evaporate, the stent is dipped briefly in fresh solvent to remove excess surface bound drug. A solution of polymer, chosen from any identified in the first experimental method, is applied to the stent as detailed above. This outerlayer of polymer will act as diffusion controller for release of drug.

The quantity and type of polymers employed in the coating layer containing the pharmaceutic agent will vary depending on the release profile desired and the amount of drug employed. The product may contain blends of the same or different polymers having different molecular weights to provide the desired release profile or consistency to a given formulation.

Absorbable polymers upon contact with body fluids including blood or the like, undergoes gradual degradation (mainly through hydrolysis) with concomitant release of the dispersed drug for a sustained or extended period (as compared to the release from an isotonic saline solution). Nonabsorbable and absorbable polymers may release dispersed drug by diffusion. This can result in prolonged delivery (e.g., 1 to 2,000 hours, preferably 2 to 800 hours) of effective amounts (e.g., 0.001 µg/cm² min to 100 µg/cm² min) of the drug. The dosage can be tailored to the subject being treated, the severity of the affliction, the judgment of the prescribing physician, and the like.

Individual formulations of drugs and polymers may be tested in *in vitro* and *in vivo* models to achieve the desired drug release profiles. For example, a drug could be formulated with a polymer (or blend) coated on a stent and placed in an agitated or circulating fluid system (such as PBS 4% bovine albumin). Samples of the circulating fluid could be taken to determine the release profile (such as by HPLC). The release of a pharmaceutical compound from a stent coating into the interior wall of a lumen could be modeled in appropriate porcine system. The drug release profile could then be monitored by appropriate means such as, by taking samples at specific times and assaying the samples for drug concentration (using HPLC to detect drug concentration). Thrombus formation can be modeled in animal models using the ¹¹¹In-platelet imaging methods described by Hanson and Harker, Proc. Natl. Acad. Sci. USA 85:3184-3188 (1988). Following this or similar procedures, those skilled in the art will be able to formulate a variety of stent coating formulations.

### Drugs To Be Delivered

The coatings can be used to deliver therapeutic and pharmaceutic agents and in particular, hydrophobic analogs or prodrugs of agents including, but not limited to: antiproliferative/antimitotic agents including natural products such as vinca alkaloids (e.g., coclchicines, vinblastine, vincristine, and vinorelbine), taxanes (e.g., paclitaxel, docetaxel), epothilones, combretastatins, epidipodophyllotoxins (e.g., etoposide, teniposide), camptothecins, antibiotics (e.g., dactinomycin (actinomycin D) daunorubicin, doxorubicin and idarubicin), geldanamycin antibiotics (e.g., geldanamycin, 17AAG), anthracyclines, mitoxantrone, bleomycins, plicamycin (mithramycin) and mitomycin, enzymes (e.g., L-asparaginase); antiproliferative/antimitotic alkylating agents, for example, nitrogen mustards (e.g., mechlorethamine, cyclophosphamide and analogs, melphalan, chlorambucil), ethylenimines and methylmelamines (e.g., hexamethylmelamine and thiotepa), alkyl sulfonates busulfan, nitrosoureas (e.g., carmustine (BCNU) and analogs, streptozocin), trazenes dacarbazinine (DTIC); antiproliferative/antimitotic antimetabolites such as folic acid analogs (methotrexate), pyrimidine analogs (e.g., fluorouracil, floxuridine, and cytarabine), purine analogs and related inhibitors (e.g., mercaptopurine, thioguanine, pentostatin and 2 chlorodeoxyadenosine(cladribine)); EGF inhibitors, platinum coordination complexes (e.g., cisplatin, carboplatin), procarbazine, hydroxyurea, mitotane, aminoglutethimide; hormones (e.g., estrogen); anticoaglants (e.g., heparin, synthetic heparin salts and other inhibitors of thrombin); fibrinolytic agents (e.g., tissue plasminogen activator, streptokinase and urokinase); antiplatelet: (e.g., aspirin, dipyridamole, ticlopidine, clopidogrel, abciximab); antimigratory; antisecretory (e.g., breveldin); antiinflammatory: such as adrenocortical steroids (cortisol, cortisone, fludrocortisone, prednisone, prednisolone, 6.α-methylprednisolone, triamcinolone, betamethasone, and dexamethasone), non steroidal agents (salicylic acid derivatives e.g., aspirin; para aminophenol derivatives e.g., acetominophen; indole and indene acetic acids (e.g., indomethacin, sulindac, and etodalac), heteroaryl acetic acids (e.g., tolmetin, diclofenac, and ketorolac), arylpropionic acids (e.g., ibuprofen and derivatives), anthranilic acids (e.g., mefenamic acid, and meclofenamic acid), enolic acids (piroxicam, tenoxicam, phenylbutazone, and oxyphenthatrazone), nabumetone, gold compounds (e.g., auranofin, aurothioglucose, gold sodium thiomalate); immunosuppressive: (e.g., cyclosporine, tacrolimus (FK 506), sirolimus (rapamycin), azathioprine, mycophenolate mofetil); angiogenic: vascular endothelial growth factor (VEGF), fibroblast growth factor (FGF); nitric oxide donors; anti-sense oligonucleotides and combinations thereof.

The following examples further illustrate the invention but, of course, should not be construed as in any way limiting its scope.

### EXAMPLE 1

### TAXANES AND ANALOGS

The following Taxanes and analogs are invention compounds suitable for use on a stent or other medical device.

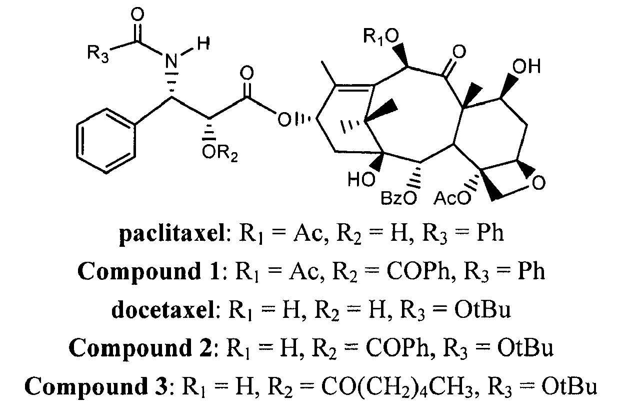

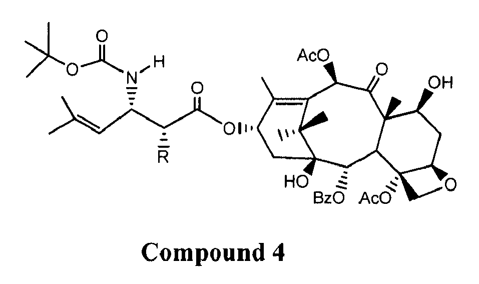

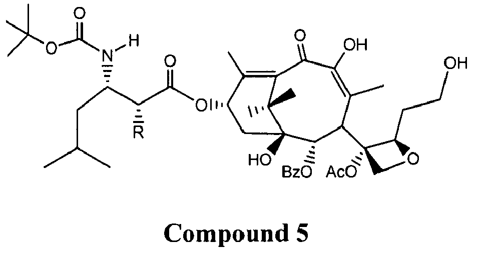

In addition to compounds 4 and 5, analogs thereof are provided by the invention in which R may be OH, OCOPh or OCO(CH₂)₄CH₃.

### EXAMPLE 2

### PREPARATION OF 2' BENZOYL DOCETAXEL (2)

An example of synthesis of one of the invention taxanes is provided herein. To a solution of docetaxel (201 mg, 0.25 mmol) in methylene chloride (6 mL) was added triethylamine (42 µL, 0.30 mmol), followed by benzoyl chloride (29 µL, 0.25 mmol) at 0 °C. The mixture was stirred at room temperature for 2 h, upon which TLC indicated the disappearance of the starting material. After quenching the reaction by adding saturated sodium bicarbonate solution, the mixture was extracted with ethyl ether. The organic layers were washed by brine, dried over anhydrous magnesium sulfate, filtered, and concentrated *in vacuo.* The residue was purified by flash silica gel column chromatography (hexane : DCM, 1 : 1) to afford the product as a white foam (181 mg, 80%). 1H NMR (CDCl3, 500 MHz): δ 8.10 (d, J = 7.5 Hz, 2H), 7.98 (d, J = 7.6 Hz, 2H), 7.61 (t, J = 7.4 Hz, 1H), 7.50 (t, J = 7.9 Hz, 2H), 7.45 (t, J = 7.8 Hz, 2H), 7.41 7.36 (m, 4H), 7.29 7.26 (m, 1H), 6.25 (t, J = 8.6 Hz, 1H), 5.67 (d, J = 7.0 Hz, 1H), 5.58-5.45 (m, 3H), 5.22 (s, 1H), 4.94 (dd, J = 9.6, 1.9 Hz, 1H), 4.31 (d, J = 8.5 Hz, 1H), 4.27 (dd, J = 10.9, 6.6 Hz, 1H), 4.19 (s, 1H), 4.18 (d, J = 8.5 Hz, 1 H), 3.93 (d, J = 6.9 Hz, 1H), 2.60-2.58 (m, 1H), 2.43 (s, 3H), 2.32-2.25 (m, 1H), 2.17 (s, 3H), 2.15-2.05 (m, 1H), 1.98 (s, 3H), 1.88-1.80 (m, 1H), 1.75 (s, 3H), 1.34 (s, 9H), 1.22 (s, 3H), 1.11 (s, 3H). ESI MS: calcd. for C50H57NO15Na (M+Na)+: 934. Found: 934.

### EXAMPLE 3

### CAMPTOTHECIN AND ANALOGS

The following camptothecins and analogs are invention compounds suitable for use on a stent or other medical device. Also incorporated by reference are those analogs described in U.S. Provisional Patent Applications 60/532,231 and 60/531,941 and PCT Patent Applications PCT/US04/43719 and PCT/US04/43978.

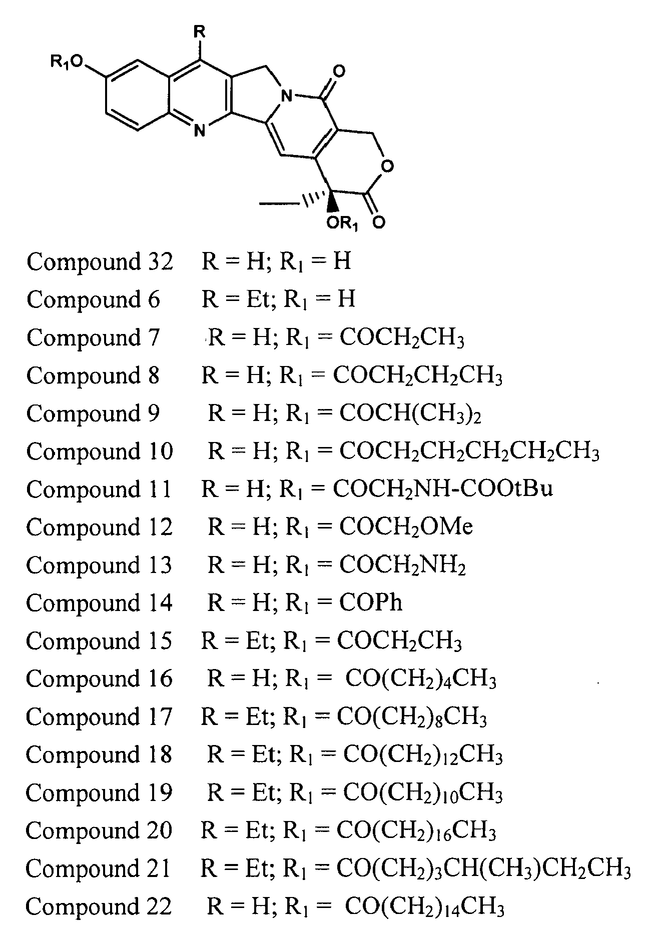

### EXAMPLE 4

### PREPARATION OF CAMPTOTHECIN 10,20-D I OHEXONATE (10)

An example of synthesis of one of the inventive camptothecins is provided herein. To a round bottomed flask was added 10 hydroxycamptothecin (1.8 g, 4.94 mmol), hexanoic anhydride (50 mL), and a few drops of concentrated sulfuric acid under stirring at room temperature. The reaction mixture was stirred at about 100 °C for overnight (∼15 h). After cooling to room temperature, the mixture was poured into 300 mL petroleum ether portion by portion while stirring. After the mixture was stirred for about 45 min, the precipitates were collected by filtration and partitioned with dichloromethane and 5% NaHCO3. The organic layer was washed with brine, dried over anhydrous Na2SO4, filtered and concentrated in vacuo. The residue was purified by flash silica gel column chromatography eluted with tetrahydrofuran/dichloromethane (5 10%) to afford the desired product as a white solid (2.4 g, 86%). 1H NMR (500 MHz, CDCl3) 0.83 (t, J=7.5 Hz, 3H), 0.92 (t, J=7.0 Hz, 3H), 0.96 (t, J=7.5 Hz, 3H), 1.31 (m, 4H), 1.40 (m, 4H), 1.64 (m, 2H), 1.79 (m, 2H), 2.13 (dq, J=14.0, 7.5 Hz, 1H), 2.26 (dq, J=14.0, 7.5 Hz, 1H), 2.48 - 2.39 (m, 2H), 2.63 (t, J=7.5 Hz, 2H), 5.25 (d, J = 3.3 Hz, 2H), 5.38 (d, J=17.2, 1H), 5.64 (d, J=17.2, 1H), 7.18 (s, 1H), 7.55 (dd, J=2.5, 9.1 Hz, 1H), 7.66 (d, J=2.5 Hz, 1H), 8.18 (d, J=9.1 Hz, 1H), 8.31 (s, 1H); Anal. Calcd for (C32H36N2O7 + H)+ and (C32H36N2O7 + Na)+: 561 and 583. Found: 561 and 583.

### EXAMPLE 5

### RAPAMYCIN AND ANALOGS

The following rapamycins and analogs are invention compounds suitable for use on a stent or other medical device.

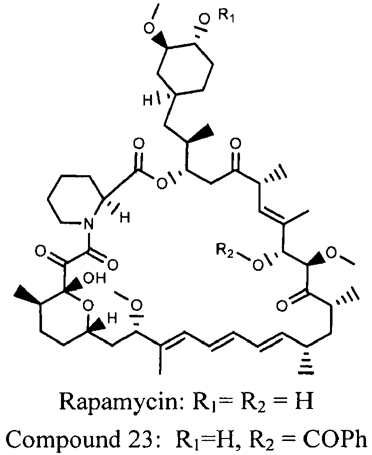

### EXAMPLE 6

### COLCHICINE AND ANALOGS

The medical device of the invention includes colchicine analogs thereon. Most preferred are dimeric structures. The following dimers are invention compounds suitable for use on a stent or other medical device.

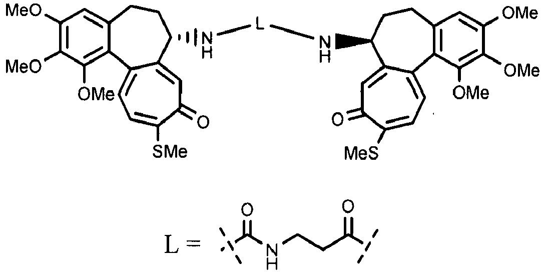

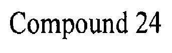

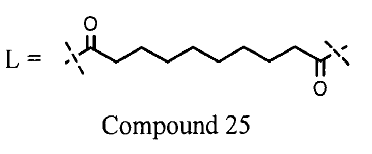

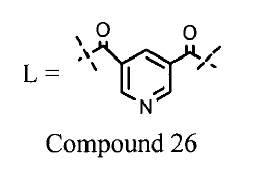

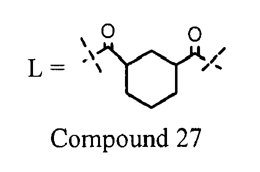

### EXAMPLE 7

### GELDANAMYCIN AND ANALOGS

The following geldanamycin antibiotics, geladanamycin and analogs are invention compounds suitable for use on a stent or other medical device. Also incorporated by reference are those compounds disclosed in the publication by Tian et al. (Bioorganic and Medicinal Chemistry 2004, 12, 5317-5329).

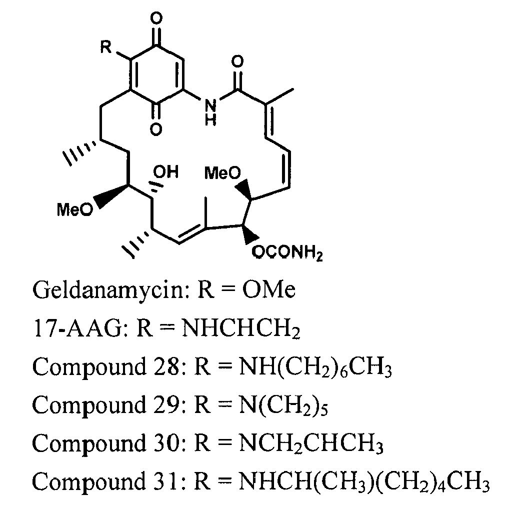

### EXAMPLE 8

### PREPARATION OF

### 17-METHYLAZIRIDINYL-17-DEMETHOXYGELDANAMYCIN (30)

An example of synthesis of one of the invention geldanamycins is provided herein. To a flame-dried three neck flask was added geldanamycin (425 mg, 0.75 mmol) and anhydrous THF (40 mL). Under an atmosphere of argon 2-methylaziridine (719 µL, 4.5 mmol) was added dropwise to the solution. The reaction mixture was stirred at room temperature for 7 h, upon which TLC indicated the disappearance of the starting material. The reaction mixture was condensed on a rotavapor to dryness. The resultant brownish oil was dissolved in 4 mL of isopropanol at 60 °C and maintained at room temperature for at least 24 h until most of the desired product recrystallized from the solvent. After careful removal of the supernatant solution via a glass pipette, the solids were washed with cold ethyl ether and dried *in vacuo* to afford the desired product (400 mg, 89.9%). ¹H NMR (CDCl₃, 500 MHz): δ 8.80 (brs, 1H), 7.27 (s, 1H), 6.93 (d, *J* = 11.0 Hz, 1H), 6.57 (t, *J* = 11.1 Hz, 1H), 5.89-5.81 (m, 2H), 5.19 (d, *J* = 4.4 Hz, 1H), 4.80 (brs, 2H), 4.32 (d, *J=* 9.6 Hz, 1H), 4.12 (s, 1H), 3.58-3.50 (m, 2H), 3.45-3.40 (m, 1H), 3.35 (s, 3H), 3.28 (s, 3H), 2.78-2.71 (m, 1H), 2.60-2.52 (m, 1H), 2.50-2.40 (m, 2H), 2.33-2.31 (m, 1H), 2.18 (d, *J* = 5.9 Hz, 1H), 2.02 (s, 3H), 1.60 (s, 3H), 1.46 (t, *J=* 5.5 Hz, 3H), 1.30-1.26 (m, 1H), 1.02-0.89 (m, 6H), 0.88 (t, *J=* 6.8 Hz, 1H). ESI-MS: calcd. for C₃₁H₄₃N₃O₈+Na (M+Na)⁺: 608. Found: 608.

### EXAMPLE 9

### PREPARATION OF COMBRETASTATIN AND ANALOGS

The following combretastatin and analogs are invention compounds suitable for use on a stent or other medical device. Combretastatin and its analogs were synthesized. Below are structures of the compounds synthesized. Also incorporated by reference are those compounds disclosed in the publication by Keira Gaukronger et al. (The Journal of Organic Chemistry 2001, 66, 8135-8138).

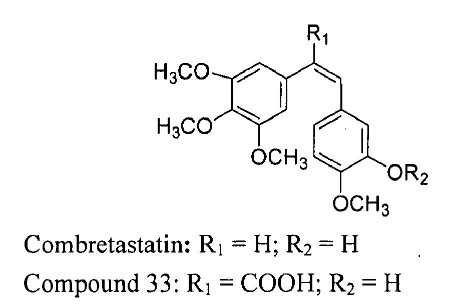

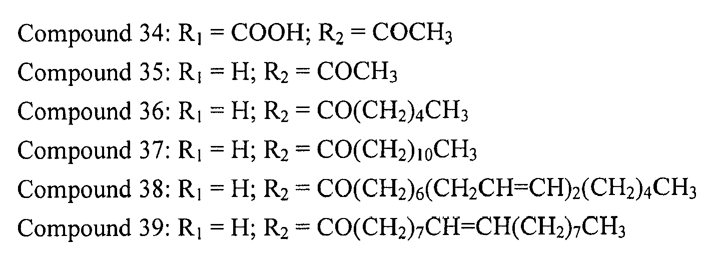

### EXAMPLE 10

### PREPARATION OF COMBRETASTATIN-HEXANOYL ESTER (36)

An example of synthesis of one of the invention combretastatins is provided herein. To a flame-dried round bottom flask was added combretastatin (0.19 g, 0.60 mmol) and anhydrous dichloromethane (10 mL). Triethylamine (0.21 mL, 1.51 mmol) was added and the mixture was cooled to 0 °C under an atmosphere of argon. Hexanoyl chloride (0.13 mL, 0.91 mmol) was added and the mixture was stirred at 0 °C to room temperature overnight, upon witch TLC indicated the disappearance of the starting material. Ethyl acetate was added and the mixture was washed by 5% NaHCO₃, water, dried (Na₂SO4) and concentrated to leave a residue. The residue was purified by column chromatography on silica gel (eluting solvent: 0-20% ethyl acetate in hexanes) yielding compound 36 as an oil (0.24 g, 97%): ¹H NMR (500 MHz, CDCl₃) δ 7.11 (1H, dd, J = 8.51, J = 2.13, H6'), 7.00 (1H, d, J = 2.09, H2'), 6.84 (1H, d, J = 8.51, H5'), 6.51 (2H, s, H2, 6), 6.47 (1H, d, J = 12.23, H1a), 6.44 (1H, d, J = 12.22, H1a'), 3.83 (3H, s, 4-OCH₃), 3.80 (3H, s, 4'-OCH₃), 3.71 (6H, s, 3,5-OCH₃), 2.52 (2H, t, J =7.49, CH₂CO), 1.73 (2H, m, CH₂CH₂CO), 1.37 (4H, m, 2xCH₂), 0.91 (3H, t, J = 6.94, CH₃); ESI-MS: calcd for (C24H30O6Na) 437, found 437 (MNa⁺); HPLC retention time 28.512 minute, 99.63%.

### EXAMPLE 11

### INCREASED HYDROPHOBICITY OF INVENTION COMPOUNDS

To increase the penetration and retention of a drug released from a device such as a stent into the vascular wall or wall of other vessel or tissue, several drugs were modified to increase their hydrophobicity. Increased hydrophobicity results in stronger binding to lipidic components of cell walls and other components of the target tissue with resultant greater retention and therefore, prolonged and improved activity in, for example, the suppression or prevention of proliferation and migration of cells involved in restenosis following balloon angioplastly and stenting of blood vessels. Hydrophobicity of the invention compounds was measured by relative elution time from a C18 HPLC column using Acetonitrile (ACN)/water as the mobile phase. The longer the elution time, the more hydrophobic the compound. Also LogP for the compounds were calculated - higher the value, more hydrophobic the compound. The table below shows elution time and logP for invention compounds.

| **Parent compound and hydrophobic analogs** | **HPLC retention time (min)** | **HPLC retention time (min)** | **LogP***** |
|---|---|---|---|
| Condition | Condition 1* | Condition 2* | |
| | | | |

| **Taxanes and analogs** | | | |
|---|---|---|---|
| Paclitaxel | | 10.5 | 4.55 |
| Docetaxel | | 9.0 | 4.20 |
| Compound 1 | | 22.7 | 6.90 |
| Compound 2 | | 22.3 | 6.55 |
| Compound 3 | | 25.6 | 6.44 |
| Compound 4 | | 23.2 | 3.19 |
| Compound 5 | | 11.0** | 4.08 |
| | | | |

| **Camptochecins and analogs** | | | |
|---|---|---|---|
| Compound 32 | 6.1 | 1.9 | 1.59 |
| Compound 7 | 19.8 | 6.1** | 3.24 |
| Compound 8 | 21.9 | | 4.11 |
| Compound 9 | 22.0 | | 4.78 |
| Compound 10 | 25.3 | | 5.85 |
| Compound 11 | 20.9 | | 3.61 |
| Compound 12 | 14.1 | | 1.42 |
| Compound 13 | 6.1 | | -0.41 |
| Compound 14 | 22.9 | | 6.06 |
| Compound 6 | 7.8 | 2.2 | 2.26 |
| Compound 15 | 20.8 | 9.3** | 3.91 |
| Compound 16 | | | 6.52 |
| Compound 17 | | 31.2 | 9.99 |
| Compound 18 | | 31.2 | 11.24 |
| Compound 19 | | 31.2 | 10.55 |
| Compound 20 | | 30.6 | 11.94 |
| Compound 21 | | 40.2 | 7.98 |
| Compound 22 | | 30.5 | 11.65 |
| | | | |

| **Rapamycin and analogs** | | | |
|---|---|---|---|
| Rapamycin | | 25.6* | 5.93 |
| Compound 23 (Benzoyl rapamycin) | | 32.6* | 8.50 |
| | | | |

| **Colchicines and Analogs** | | | |
|---|---|---|---|
| Compound 24 | | 12.8 | 5.93 |
| Compound 25 | | 19.3 | 8.5 |
| Compound 26 | | 11.6 | 5.75 |
| Compound 27 | | 9.1 | 6.74 |
| | | | |

| **Geldanamycins and Analogs** | | | |
|---|---|---|---|
| Geldanamycin | | 10.8 | -0.6 |
| 17AAG | | 14.9 | 0.01 |
| Compound 28 | | 14.9 | 1.67 |
| Compound 29 | | 23.9 | -0.13 |
| Compound 30 | | 11.1/11.6 | -0.37 |
| Compound 31 | | 30.3/30.7 | 1.57 |

| | | | |
|---|---|---|---|
| Condition 1* Mobile phase - A: Acetonitrile B: (30% acetonitrile : 70% 75 mM ammonium acetate buffer (pH 6.4)) with 5mM TBAP A/B (0:100) from 0 to 6 minutes; to A/B (100:0) from 6 to 20 minutes; to A/B (0:100) at 25 minutes Flow rate - 0.8 mL/min Column temperature - 35°C Condition 2* Mobile phase - A: Acetonitrile B: Water A/B (50:50) from 0 to 10 minutes; to A/B (90:10) from 10 to 30 minutes; to A/B (50:50) at 40 minutes Flow rate - 1 mL/min Column temperature - 35°C **Column temperature was set at 70°C ***LogP were calculated with Molinspiration Property Calculation Services at www.molinspiration.com. And the LogP of geldanamycin and analogs were calculated with ChemDraw Ultra of CambridgeSoft Corporation | | | |

The retention/elution time of the invention compounds clearly show an increase in hydrophobicity compared to parent compounds such as paclitaxel, docetaxel, camptothecin, rapamycin, colchicine and geldanamycin.

### EXAMPLE 12

### CYTOTOXIC ACTIVITY OF INVENTION HYDROPHOBIC COMPOUNDS ON MX-1 MAMMARY TUMOR CELLS IN CULTURE

| **Parent compound and hydrophobic analogs** | **Cytotoxicity on MX-1, IC₅₀ (uM)****** |
|---|---|
| | |

| **Taxanes and analogs** | |
|---|---|
| Paclitaxel | 73, 4, (0.5) |
| Docetaxel | 48, 8 |
| Compound 1 | 30, (0.9) |
| Compound 2 | 38, (1.3) |
| Compound 3 | 2 |
| | |

| **Camptothecins and analogs** | |
|---|---|
| Compound 32 | 13 |
| Compound 7 | 45 |
| Compound 8 | 152 |
| Compound 9 | 23 |
| Compound 10 | 8 |
| Compound 11 | 221 |
| Compound 12 | 372 |
| Compound 13 | 242 |
| Compound 14 | 740 |
| Compound 6 | 267 |
| Compound 15 | 28 |
| Compound 16 | 4 |
| Compound 17 | 2000 |
| Compound 18 | Inactive |
| Compound 19 | Inactive |
| Compound 20 | Inactive |
| Compound 21 | 295 |
| Compound 22 | Inactive |
| | |

| **Rapamycin and analogs** | |
|---|---|
| Rapamycin | 27, (5) |
| Compound 23 (Benzoyl rapamycin) | 10 |
| | |

| **Colchicines and analogs** | |
|---|---|
| Compound 24 | 37, (21) |
| Compound 25 | 155, (39) |
| Compound 26 | 361, (83) |

| **Geldanamycins and analogs** | |
|---|---|
| Geldanamycin | 0.5 |
| 17AAG | 3 |
| Compound 28 | 20 |
| Compound 30 | 0.3 |
| Compound 31 | 12.8 |

| | |
|---|---|
| Data in ( ) indicate nanoparticle albumin versions if the drugs. | |

### EXAMPLE 13

### BINDING OF COMPOUNDS TO ALBUMIN AS A SURROGATE FOR PERSISTENCE IN TISSUE

The K_{D} for binding of invention compounds to the protein albumin was used as an indicator of the binding affinity of invention compounds to proteins and cellular components (see table below). A smaller number indicates a greater binding affinity.

| **Parent compound and hydrophobic analogs** | **Albumin Binding, K_{D} (uM)** |
|---|---|
| | |

| **Taxanes and Analogs** | |
|---|---|
| Paclitaxel | 39.8 |
| Docetaxel | 5.45 |
| Compound 1 | 103 |
| Compound 2 | 8.8 |
| Compound 4 | 85 |
| Compound 5 | 279 |
| | |

| **Camptothecins** | |
|---|---|
| Compound 32 | 1201 |
| Compound 6 | 484 |

| **Rapamycins and analogs** | |
|---|---|
| Rapamycin | 102 |
| | |

| **Colchicines and Analogs** | |
|---|---|
| Compound 24 | 109 |
| Compound 25 | 30 |
| Compound 26 | 28 |
| Compound 27 | 52 |

### EXAMPLE 14

### COATING OF DRUGS ON DEVICES USING POLYMERS

Solutions of hydrophobic invention drugs, such as the geldanamycin analogs, 17-AAG, rapamycin analog, taxane analogs, colchicine analogs, or camptothecin analog were prepared in acetone or methylene chloride. This solution was mixed with the polymer carrier solution to give final concentration range 0.001 wt% to 30 wt% of drug. The polymer/drug mixture was applied to the surfaces of the stentby either dip-coating and the solvent allowed to evaporate to leave a film with entrapped drug within the polymer on the stent.

### EXAMPLE 15

### COATING OF DRUGS ON DEVICES WITHOUT POLYMERS

A medical device, for example, an intravascular stent, was dipped into a solution of geldanamycin analogs, 17-AAG, rapamycin analog, taxane analogs, or camptothecin analog, in a range of 0.001- wt % to saturated, in organic solvent, such as, acetone, methylene chloride, ethyl acetate or other volatile solvent for sufficient time to allow solution to fully contact the device. The device was removed, thereafter and the solvent evaporated. Optionally, after the solvent was evaporated, a solution of a polymer, chosen from any identified above, could be applied applied to the stent as detailed above. This outerlayer of polymer acted as a diffusion-controller for release of the drug.

### EXAMPLE 16

### COATING USING AN ABSORBABLE POLYMER

An absorbable elastomer based on 45:55 mole percent copolymer of έ-caprolactone and glycolide, with an intrinsic viscosity of 1.58 (0.1 g/dL in hexafluoroisopropanol[HFIP] at 25 °C) was dissolved five percent (5%) by weight in acetone and separately fifteen percent (15%) by weight in 1,1,2-trichloroethane. The synthesis of the elastomer is described in U.S. Patent 5,468,253, which is incorporated herein by reference. Other suitable polymers as mentioned above could also be utilized. Gentle heating can be used to increase the dissolution rate. The high concentration coating could be formulated with or without pharmaceutical agent present. An initial primer coat of only the polymer was put on a Guidant Multilink 2.5 x 15mm stent by dip coating in the five percent (5%) solution while the stent is placed on a 0.032 inch (0.81 mm) diameter mandrel. The mandrel, with the stent on it, is removed from the dip bath and before the coating has a chance to dry the stent is moved along the length on the mandrel in one direction. This wiping motion applies high shear to the coating trapped between the stent and the mandrel. The high shear rate forces the coating out through the slots cut into the tube from which the stent is formed. This wiping action serves to force the coating out of the slots and keeps them clear. The "primed stent" is allowed to air dry at room temperature. The prime coat is about 100 micrograms of coating. After 1-2 hours of air drying, the stent is remounted on a 0.0355 inch (0.9 mm) clean mandrel and dipped into a second, concentrated coat solution. This can be drug free or can contain about six percent (6%) by weight drug in addition to about fifteen percent (15%) polymer by weight in the coating solution. The dip and wipe process is repeated. The final coated stent is air dried for 12 hours and then put in a 60 °C vacuum oven (at 30 in.Hg vacuum) for 24 hours to dry. This method provides a coated stent with about 270 micrograms of polymer and about 180 micrograms of drug.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A medical device comprising a medicament comprising rapamycin or a hydrophobic analog of rapamycin of the formula: 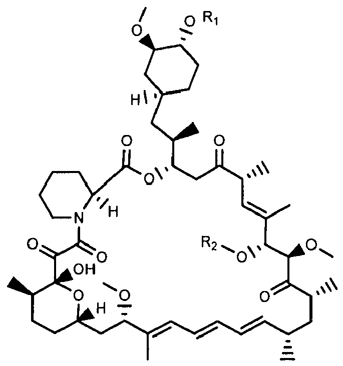 wherein, R₁ is H and R₂ is H or COPh.

2. The device of claim 1, wherein said medicament is coated on or incorporated within the body of the device.

3. The device of claim 1, wherein the medicament is incorporated onto or within the device in presence of a polymer.

4. The device of claim 2, wherein said analog is present in the coating in an amount of from about 0.0001% to about 30% by weight of said coating.

5. The device of claim 3, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.

6. The device of claim 3, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.

7. The device of claim 6, wherein said medicament is coated on or incorporate within the body of the device.

8. The device of claim 6, wherein the medicament is incorporated onto or within the device in presence of a polymer.

9. The device of claim 7, wherein said analog is present in the coating in an amount of from about 0.0001% to about 30% by weight of said coating.

10. The device of claim 8, wherein said polymer is selected from the group consisting of lactone based polyesters, lactone based copolyesters; polyanhydrides; polyaminoacids; polysaccharides; polyphosphazenes; poly(ether ester) copolymers, and blends of such polymers.

11. The device of claim 8, wherein the device is a stent, and wherein the polymer is selected from the group consisting of polydimethylsiloxane; poly(ethylene)vinylacetate; poly(hydroxy)ethylmethylmethacrylate, polyvinyl pyrrolidone; polytetrafluoroethylene; and cellulose esters.
